Europäisches Patentamt
European Patent Office
Office européen des brevets

(11) Publication number: **0 083 185**
**A1**

# (12) EUROPEAN PATENT APPLICATION

(21) Application number: 82306782.2

(22) Date of filing: 20.12.82

(51) Int. Cl.³: C 07 C 69/96
C 08 J 7/10

(30) Priority: 30.12.81 US 335772

(43) Date of publication of application: 06.07.83 Bulletin 83/27

(84) Designated Contracting States: BE DE FR GB IT NL

(71) Applicant: MOBIL OIL CORPORATION
150 East 42nd Street
New York New York 10017(US)

(72) Inventor: Chung-Pung Chen, Albert
14 Vaux Hall Road
East Brunswick New Jersey 08816(US)

(74) Representative: West, Alan Harry et al,
Mobil Court 3 Clements Inn
London WC2A 2EB(GB)

(54) **Carbonate-polyacrylates, radiation curable coating compositions and method of curing.**

(57) Carbonate polyacrylate monomers having the formula

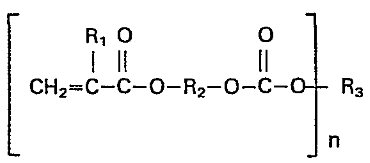

(I)

or

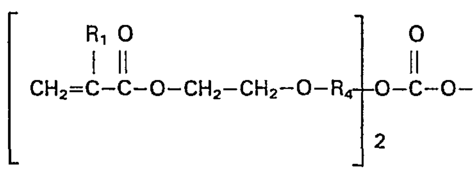

(II)

in which $R_1$ is H or alkyl;
$R_2$ is an alkylene, oxyalkylene or polyoxyalkylene;
$R_3$ is an aliphatic or aromatic polyvalent radical; and n is 2 to 6. These monomers are particularly suitable for the use in radiation curable coatings cured by ultraviolet or electron beam radiation.

EP 0 083 185 A1

## CARBONATE-POLYACRYLATES, RADIATION CURABLE COATING COMPOSITIONS AND METHOD OF CURING

This application relates to novel carbonate-polyacrylate or poly-(alkylacrylate) monomers which are suitable for radiation curable coatings, particularly for curing by ultraviolet or electron beam. These compositions may also be employed as reactive diluents for other acrylate and alkylacrylate resinous materials to produce strong and useful coatings.

The carbonate-polyacrylate and carbonate poly-(alkylacrylate) monomers of this invention have the formula

$$\left[ CH_2{=}\overset{R_1}{\overset{|}{C}}-\overset{O}{\overset{\|}{C}}-O-R_2-O-\overset{O}{\overset{\|}{C}}-O- \right]_n R_3 \qquad (I)$$

or

$$\left[ CH_2{=}\overset{R_1}{\overset{|}{C}}-\overset{O}{\overset{\|}{C}}-O-CH_2-CH_2-O-R_4- \right]_2 O-\overset{O}{\overset{\|}{C}}-O- \qquad (II)$$

in which

$R_1$ is H or $C_1$-$C_4$ alkyl, preferably H or $CH_3$;

$R_2$ and $R_4$ are alkylene radicals of 2 to 6 carbon atoms which may contain oxygen in the alkylene chain to form an alkylene ether or poly(alkylene ether) radicals having 2 to 6, preferably 2 or 3 carbon atoms in the alkylene moiety;

$R_3$ is a polyvalent radical derived from alkyl of 1 to 12 carbon atoms, alkylene ether or polyalkylene ether containing 1 to 6 carbon atoms in the alkylene group, cycloalkyl, substituted cycloalkyl of 5 to 12 carbon atoms, phenyl or substituted phenyl; and n is 2 to 6.

Preferred cycloalkyl radicals are cyclopentyl and particularly cyclohexyl which can be substituted, for example by alkyl groups.

The phenyl radical can be substituted with non-interfering substituents such as $C_1$-$C_4$ alkyl; $C_1$-$C_4$ alkoxy halogen, particularly chloro, cyano, nitro and the like.

Particularly suitable $R_3$ moieties are ethylene, propylene poly(ethylene glycol) and poly(propyleneglycol) radicals.

The acrylates and methacrylates in which $R_1$ is H or methyl, respectively, are preferred.

The carbonate-polyacrylate compounds of this invention can be made by methods which are in themselves known. For example, the polycarbonates of formula I can be prepared by reacting a suitable hydroxy substituted acrylate with phosgene to form the chloroformate which can then be reacted with the appropriate polyol containing the $R_3$ moiety. Alternatively, diphenyl-carbonate can be substituted for phosgene. Analogously, a polyhydroxy compound containing the $R_3$ moiety can be converted to the chloroformate or phenylcarbonate by reaction with phosgene or diphenylcarbonate and this intermediate reacted with the desired hydroxy acrylate.

The compounds of formula II are prepared in a manner also known for example as shown in U.S. Patent 3,619,260. Briefly, two or more moles of the desired hydroxyacrylate are reacted with a mole of phosgene preferably in the presence of an acid acceptor such as a tertiary amine. An acid acceptor is desirable in any of the described preparative reactions involving phosgene.

The carbonate polyacrylate compounds of this invention and mixtures thereof are useful in a variety of applications which are in themselves known for other monomers, particularly acrylate monomers. These compounds and mixtures are especially suitable for use alone, or in mixtures with other monomers as radiation curable coatings.

Curing of the compositions containing the carbonate polyacrylate compounds disclosed herein can be accomplished with radiation, advantageously with electron beam or ultraviolet radiation. When ultraviolet radiation is utilized incorporation of a photosensitizer in the composition is desirable. Typical

photosensitizers are benzophenone and benzoin ethers such as the ethyl or butyl ethers, but these are merely illustrative as many photosensitizers are known in the art and are commercially available.

The compositions containing the carbonate-polyacrylate monomers of this invention are suitable for coating a variety of substrates including metals, wood, paper and plastics.

The invention is illustrated by the following example in which the parts are by weight.

## EXAMPLE

A solution was prepared by dissolving 46.4 parts of 2-hydroxyethyl acrylate, 38.0 parts of pyridine, 0.5 parts of 4-dimethylaminopyridine, 0.02 parts of 4,4'-methylenebis (2,6-di-t-butylphenol) and 200 parts of dry toluene. To it was added 46.2 parts of diethylene glycol bis (chloroformate) in 20 minutes. The reactants were then heated at 70°C for 2 hours. After cooling, solid precipitates were filtered and the solvents removed by vacuum distillation.

The reaction product is radiation curable alone or in mixtures with other radiation curable monomers to yield a useful coating.

CLAIMS:

1. A compound of the formula

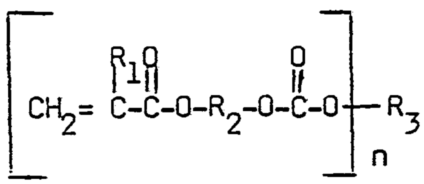

in which

$R_1$ is H or alkyl of 1 to 4 carbons;

$R_2$ is an alkylene, an alkylene ether or a poly(alkylene ether) radical containing 2 to 6 carbon atoms in the alkylene group; and

$R_3$ is alkyl of 1 to 12 carbon atoms, a radical derived from alkylene ether or polyalkylene ether containing 2 to 6 carbon atoms in the alkylene group, cycloalkyl or substituted cycloalkyl of 5 to 12 carbon atoms, phenyl or substituted phenyl; and

n is 2 to 6.

2. The compound of claim 1 in which $R_1$ is H.

3. The compound of claim 1 in which $R_1$ is $-CH_3$.

4. The compound of claim 1 in which $R_2$ is $-CH_2-CH_2-$.

5. The compound of claim 1 in which $R_3$ is an alkyl radical of 1 to 8 carbon atoms.

6. The compound of claim 1 in which $R_3$ is $—(CH_2-CH_2O)_mCH_2-CH_2-$; and m is an integer from 1 to 12.

7. The compound of claim 1 in which $R_3$ is

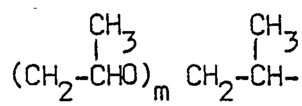

and m is an integer from 1 to 12.

8. A compound of the formula

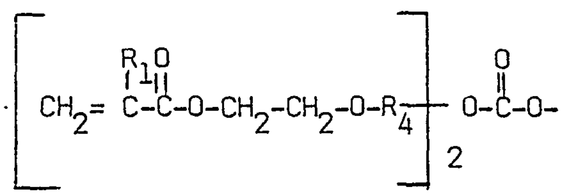

$R_1$ is H or alkyl of 1 to 4 carbon atoms;
$R_4$ is alkylene of 2 to 6 carbon atoms,

$(CH_2-CH_2-O)_m\ CH_2-CH_2-$ or

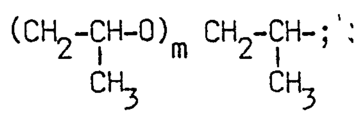

in which m is 1 to 12.

9. A composition comprising the compound of claims 1 or 8 and up to 99 weight percent of the composition as a different radiation curable monomer.

10. A method of preparing a radiation curable coating comprising applying a composition including the compound of claims 1 or 8 to a substrate and curing the composition to a film with radiation.

9619N

European Patent Office

# EUROPEAN SEARCH REPORT

0083185

Application number

EP 82 30 6782

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | US-A-3 785 850 (G.M.PARKER) *Column 1, lines 21-43; column 2, lines 1-30; column 3, lines 10-28; column 4, lines 21-30; column 5, examples 1,3* | 1-10 | C 07 C 69/96<br>C 08 J 7/10 |
| X | US-A-3 716 571 (A.A.BERLIN et al.) *Column 1, line 60 - column 2, line 7; column 2, lines 20-36; column 3, example 1* | 1-6,8,9 | |
| X | DE-A-2 026 311 (PPG INDUSTRIES) *Page 2, paragraph 1; page 3, paragraphs 1-3; page 6, paragraph 3 - page 7, paragraph 2; page 9, paragraph 2; page 13, example 7; page 14, example 8; page 15, example 10* | 1-10 | TECHNICAL FIELDS SEARCHED (Int. Cl. 3)<br>C 07 C 69/00 |

The present search report has been drawn up for all claims

| Place of search | Date of completion of the search | Examiner |
|---|---|---|
| THE HAGUE | 14-03-1983 | KINZINGER J.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82